Europäisches Patentamt

European Patent Office

Office européen des brevets

(11) Publication number: **0 296 248**
**A1**

(12) # EUROPEAN PATENT APPLICATION

published in accordance with Art. 158(3) EPC

(21) Application number: 88900578.1

(22) Date of filing: 24.12.87

Data of the international application taken as a basis:

(86) International application number:
PCT/JP87/01026

(87) International publication number:
WO88/04941 (14.07.88 88/15)

(51) Int. Cl.³: **A 61 N 1/00**

(30) Priority: 24.12.86 JP 202212/86 U

(43) Date of publication of application:
28.12.88 Bulletin 88/52

(84) Designated Contracting States:
FR GB

(71) Applicant: TOHOKU RICOH CO., LTD.
3-1, Azashinmeido Oazanakanomyo Shibatamachi
Shibata-gun Miyagi-ken 989-16(JP)

(71) Applicant: FURUKAWA CO., LTD.
6-1, Marunouchi 2-chome Chiyoda-ku
Tokyo 100(JP)

(72) Inventor: KAMIYAMA, Junichi
5-14, Midorigahama
Chigasaki-shi Kanagawa-ken 253(JP)

(72) Inventor: TSUBAKI, Yoshiharu
14-10-702, Kano 3-chome Sendai-shi
Miyagi-ken 982(JP)

(72) Inventor: HIRATSUKA, Takefumi
7-5, Kitafunaoka 3 Shibata-machi
Shibata-gun Miyagi-ken 989-16(JP)

(72) Inventor: ISHIGURO, Saburo
4-21, Hamadayama 4
Suginami-ku Tokyo 168(JP)

(72) Inventor: TANABE, Makoto
1184, Ochikawa
Tama-shi Tokyo 206(JP)

(72) Inventor: IWATA, Tetsuhiro
50, Nukari Kamiyoshima Yoshima-machi
Iwaki-shi Fukushima-ken, 970-11(JP)

(74) Representative: Cross, Rupert Edward Blount et al,
BOULT, WADE & TENNANT 27 Furnival Street
London EC4A 1PQ(GB)

(54) SEMICONDUCTOR THERAPEUTIC DEVICE.

(57) A semi-conductor therapeutic device is formed by depositing a semiconductor cover film made of selenium or its alloy on the surface of a substrate having a suitable size. When this therapeutic device is bonded to the affected part or an effective spot for remedy of the body by use of an adhesive tape, the flow of electrons is generated inside the body due to the electron characteristics of the semiconductor and the potential inside the body can be normalized so that muscle pain, stiff shoulders, and the like can be cured.

# FIG.9

SPECIFICATION

Description:

Semiconductor Medical Treatment Device

Field of the Invention:

The present invention relates to a semiconductor medical treatment device which is intended for use in curing muscle pain, stiffness in the shoulder and other painful conditions in the human body.

Background of the Art:

Abnormal distribution of electric potential in the human body is considered as being one of the causes of conditions such as muscle pain, stiffness in the shoulder and so forth. In order to cure such conditions, medical treatment devices have been heretofore used which make use of a small magnet or a piece of germanium. The small magnet or piece of germanium, which is suitably fixed to the body of the patient by, for example, adhesion, interacts with electrons in the human body so as to restore the normal distribution of the electric potential, thereby reducing the muscle pain or stiffness in the shoulder.

These known medical treatment devices making use of a small magnet or a piece or germanium, however, do not provide any appreciable effect on certain types of muscle pain, and are further unsatisfactory in that they fail to provide an immediate or long-lasting effect.

- 1 -

Disclosure of the Invention:

Accordingly, an object of the present invention is to provide a semiconductor medical treatment device which is used in contact with the painful portion of the patient's body and which provide an immediate and long-lasting effect.

To this end, according to the present invention, there is provided a semiconductor medical treatment device comprising a substrate made from a material selected from a group consisting of glasses, ceramics, plastics and metals, and a semiconductor film of selenium or a selenium alloy formed on a surface of the substrate. The medical treatment device of the invention is made to adhere to the painful portion or curing spot (tsubo) of the patient's body by means of adhesive tape. The electronic physical properties of the semiconductor produce flows of electrons in the patient's body so as to restore the normal electric potential distribution in the body.

Brief Description of the Drawings:

Figs. 1, 2 and 3 are perspective views of various forms of the semiconductor medical treatment device constructed in accordance with the present invention;

Figs. 4, 5 and 6 are cross-sectional views of the semiconductor medical treatment devices shown in Figs. 1, 2 and 3, respectively;

Fig. 7 is a cross-sectional view of another embodiment of the semiconductor medical treatment device according to the invention;

Fig. 8 is a cross-sectional view of still another embodiment of the semiconductor medical treatment device according to the present invention;

Fig. 9 is a perspective view of adhesive tape with a single piece of the semiconductor medical treatment device of the invention attached thereto; and

Fig. 10 is a perspective view of adhesive tape with a plurality of pieces of the semiconductor medical treatment device attached thereto.

The Best Mode for Carrying Out the Invention:

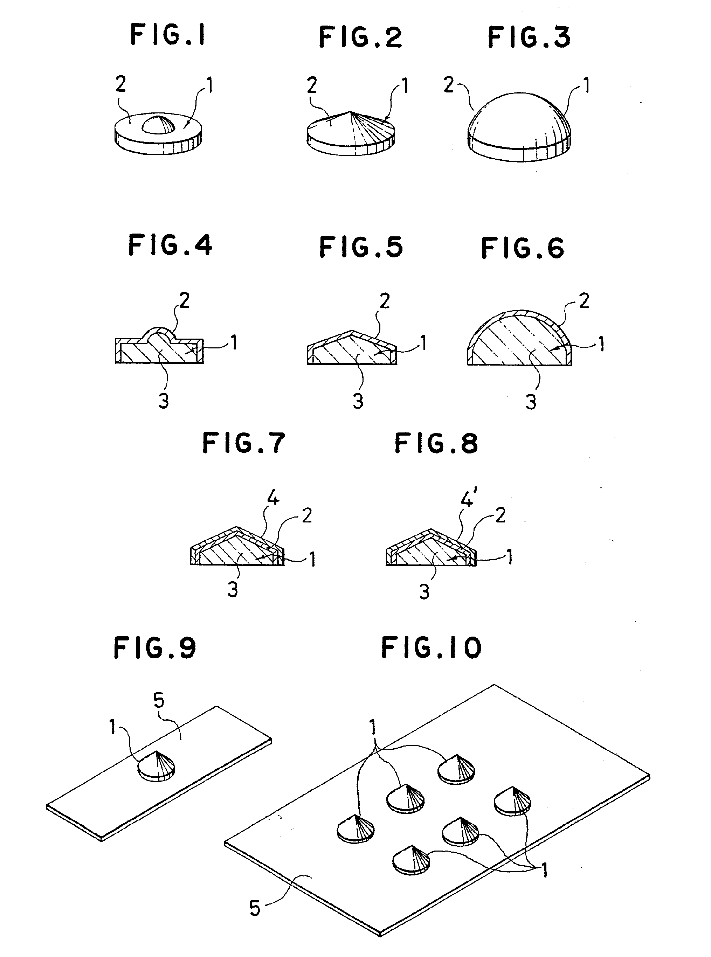

Referring to Figs. 1 to 6, a medical treatment device 1 of the present invention has a disk-shaped substrate 3 made of a material selected from a group consisting of glasses, ceramics, plastics and metals, and a semiconductor film 2 of selenium or a selenium alloy formed on the obverse surface of the substrate 3 by, for example, evaporation deposition or spattering. The substrate 3 may have a simple disk-like form or may be provided with a bean-like projection on the surface thereof as shown in Fig. 1. The substrate 3 may also have a conical projection on the disk-like base as shown in Fig. 2 or a hemispherical projection on the upper surface thereof as shown in Fig. 3.

The thickness of the semiconductor film preferably falls within a range of between 2 and 100 microns. A thickness of less than 2 microns may cause exfoliation of the semiconductor film on the top of the bean-like projection or

- 3 -

the top of the conical projection. On the other hand, it is useless to increase the thickness of the semiconductor film 2 beyond 100 microns from an electronics point of view.

The selenium alloys suitable for use as the material of the semiconductor film are a selenium-germanium alloy, a selenium-arsenic alloy or a selenium-antimony alloy.

In order to delicately control the physical electronic properties of the semiconductor film so as to widen the applicability of the medical treatment, the semiconductor medical treatment device of the present invention can have another semiconductor film such as an amorphous silicon film 4 on top of the semiconductor film 2 formed on the substrate 3. It is also possible to coat the semiconductor film 2 with a protective film 4 of a material which exhibits superior strength and which does not impair the physical electronic properties of the semiconductor film.

Examples of the manner in which the medical treatment device of the the present invention is fixed to the patient's body are shown below.

(1)    As shown in Fig. 9, a single piece of the medical treatment device 1 of the invention is attached to an adhesive surface of an adhesive sheet 5 and the adhesive tape is attached to the patient's body such that the treatment device 1 is held in contact with the painful portion of the patient's body.

(2)    As shown in Fig. 10, a plurality of pieces of the medical treatment device 1 of the present invention are attached to an adhesive tape having a relatively large area. The treatment device is then attached to the patient's body in the same way as explained in connection with Fig. 9. The semiconductor treatment device thus attached to the patient's body produces a flow of electrons in the painful portion of the patient's body due to the physical electronic properties of the semiconductor so that the normal distribution of the electrical potential is restored in the patient's body and the pain is cured.

(Example 1)

A selenium film was formed by evaporation deposition on the surface of a disk-shaped ceramic substrate having a diameter of 7 mm and a thickness of 1 mm and provided with a central bean-like projection formed thereon. The thus formed semiconductor medical treatment device was attached by means of commercially available plaster to the painful portion of the body of 12 subjects among which 6 reported that the pain was cured and 2 answered that the device was appreciably effective. Thus, the ratio between the sum of the numbers of the subjects who answered "cured" or "effective" to the total number of subjects was 67%.

(Example 2)

A germanium-selenium alloy film (selenium 95 wt % and germanium 5 % wt) of 15 microns was formed by evaporation deposition on the surface of a disk-shaped ceramic substrate having a diameter of 7 mm and a thickness of 1 mm and provided with a central bean-like projection formed thereon. The thus obtained semiconductor medical treatment device was attached to the painful portion of the body of 10 subjects. Among the 10 subjects, 6 reported that they were cured and 2 answered that the treatment device was effective. Thus, the effectiveness ratio was 80%.

(Example 3)

A selenium-arsenic alloy film (selenium 98 wt % and arsenic 2 wt %) of 5.5 microns was formed by spattering on the surface of a plastic substrate of the same size as that used in Example 1. The thus obtained semiconductor medical treatment device was attached to the painful portion of the body of 12 subjects. Among the 12 subjects, 8 answered that they were cured and 4 answered that the device was effective. Thus, the effectiveness ratio was 92%.

(Example 4)

A selenium-arsenic alloy film (selenium 98 wt % and arsenic 2 wt %) of 4.5 microns was formed by spattering on the surface of a disk-shaped glass substrate having a diameter of 4.5 mm and a height of 3.5 mm. A film of amorphous silicon was then formed by chemical vapor deposition to a thickness of 3.0 microns on the selenium-arsenic alloy film. The thus obtained semiconductor medical treatment device was attached to the painful portion of the body of 11 subjects, among which 7 answered that they were cured and 2 answered that the device was effective. Thus, the effectiveness ratio was 82%.

(Example 5)

A selenium-antimony alloy film (selenium 93 wt% and antimony 7 wt %) of 7 microns was formed by evaporation deposition on a disk-shaped aluminum substrate with a conical projection, with the substrate having a diameter of 3 mm and overall height of 3.5 mm (disk height 2 mm and cone height 1.5 mm). A coating film of 7 microns was then formed as a protective film from a resin containing phthalocyanine. The thus obtained medical treatment device was placed by adhesion on the painful portion of the body of 12 subjects, among which 6 reported that they were cured and 2 answered that the device was effective. Thus, the effectiveness ratio was 67%.

(Example 6)

Four pieces of medical treatment device as obtained in Example 2 above were attached to the surface of a hot compress plaster (7 cm x 5 mm) and the hot compress plaster was attached to the painful portion of the body of 12 subjects. Among the 12 subjects, 7 answered that they were cured and 2 reported that the device was effective. Thus, the effectiveness ratio was 75%.

As will be understood from the foregoing description, the medical treatment device of the invention, when placed by adhesion on a painful portion or curing spot (tsubo), serves to restore the normal electric potential distribution in the patient's body by virtue of the physical electronic properties of its semiconductor material, thereby curing painful conditions such as muscle pain, stiffness in the shoulder and so forth which are attributable to various causes. The conical or hemispherical shape of the medical treatment device, as well as the bean-like projection, provides a greater area of semiconductor film and, in addition, moderately stimulates the painful portion, thus ensuring a greater curing effect. Wear of the semiconductor film is avoided in the medical treatment device when a protective film is formed on the surface of the semiconductor film.

What is claimed is:

1. A semiconductor medical treatment device comprising: a substrate made of a material selected from a group consisting of glasses, ceramics, plastics and metals, and a semiconductor film formed on a surface of said substrate from selenium or a selenium alloy.

2. A semiconductor medical treatment device according to Claim 1, wherein a bean-like projection is formed on said surface of said disk-shaped substrate.

3. A semiconductor medical treatment device according to Claim 1, wherein a conical projection is formed on said surface of said disk-shaped substrate.

4. A semiconductor medical treatment device according to Claim 1, wherein a hemispherical projection is formed on said surface of said disk-shaped substrate.

5. A semiconductor medical treatment device according to Claim 1, wherein a protective film is formed on said semiconductor film.

6. A semiconductor medical treatment device according to Claim 1, wherein one or a plurality of pieces of said semiconductor medical treatment device are attached to an adhesive sheet.

FIG.1    FIG.2    FIG.3

FIG.4    FIG.5    FIG.6

FIG.7    FIG.8

FIG.9    FIG.10

## I. CLASSIFICATION OF SUBJECT MATTER (if several classification symbols apply, indicate all) 3

According to International Patent Classification (IPC) or to both National Classification and IPC

Int.Cl⁴    A61N1/00

## II. FIELDS SEARCHED

| Minimum Documentation Searched 4 | |
|---|---|
| Classification System | Classification Symbols |
| IPC | A61N1/00, A61N1/42 |

| Documentation Searched other than Minimum Documentation<br>to the Extent that such Documents are Included in the Fields Searched 5 | |
|---|---|
| Jitsuyo Shinan Koho | 1930 – 1987 |
| Kokai Jitsuyo Shinan Koho | 1971 – 1987 |

## III. DOCUMENTS CONSIDERED TO BE RELEVANT 14

| Category * | Citation of Document, 16 with indication, where appropriate, of the relevant passages 17 | Relevant to Claim No. 18 |
|---|---|---|
| A | JP, A, 58-38566 (Araki Yasuo)<br>7 March 1983 (07. 03. 83)<br>Page 1 (Family: none) | 1 |

* Special categories of cited documents: 15

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search 3 | Date of Mailing of this International Search Report 3 |
|---|---|
| March 9, 1988 (09. 03. 88) | March 22, 1988 (22. 03. 88) |
| International Searching Authority 1 | Signature of Authorized Officer 20 |
| Japanese Patent Office | |